# EUROPÄISCHE PATENTSCHRIFT

(11) **EP 0 730 830 B1**
(45) Veröffentlichungstag und Bekanntmachung des Hinweises auf die Patenterteilung: **06.02.2002**
(21) Anmeldenummer: 96103445.1
(22) Anmeldetag: 06.03.1996
(51) Int. Cl.: A23L 1/015, C02F 1/26, A61K 35/78, A62D 3/00, C11B 9/02, A23L 2/70

(54) **Verfahren zur Entfernung von unerwünschten lipophilen Verunreinigungen und/oder Rückständen, welche in Getränken oder pflanzlichen Zubereitungen enthalten sind**
Process for the removal of undesired lipophilic contaminations and/or residues which are contained in beverages or vegetable preparations
Procédé d'élimination d'impuretés et/ou résidus lipophiles indésirables présents dans des boissons ou des préparations végétales

(30) Priorität: 06.03.1995 CH 62995; 02.06.1995 CH 162195
(43) Veröffentlichungstag der Anmeldung: 11.09.1996
(73) Patentinhaber: Emil Flachsmann AG, CH-8820 Wädenswil (CH)
(72) Erfinder: Kreuter, Matthias-Heinrich, Dr., 8880 Walenstadt (CH); Steiner, Rudolf, 8806 Bäch (CH)
(74) Vertreter: Zink-Wild, Markus Peter

(56) Entgegenhaltungen:
- EP-A- 0 013 659
- EP-A- 0 080 298
- EP-A- 0 382 116
- WO-A-94/15702
- DE-B- 1 289 400
- FR-A- 2 220 292
- GB-A- 714 904
- US-A- 4 377 600
- US-A- 5 094 868
- JAOCS, Bd. 57, Nr. 9, USA, Seiten 705a-706a, XP002005067 R.JANDACEK:: "The Removal of Organic Substances from Water with Nonvolatile Edible Solvents"

## Beschreibung

Die vorliegende Erfindung betrifft ein Verfahren zur Entfernung von unerwünschten lipophilen Verunreinigungen und/oder Rückständen, welche in Getränken oder pflanzlichen Zubereitungen enthalten sind.

Organische lipophile Verbindungen werden seit Jahrzehnten als Pflanzenschutzmittel, Schädlingsbekämpfungsmittel und Pestizide verwendet.

Einige dieser hochaktiven toxischen Substanzen haben die unerwünschte Eigenschaft, sich nach Anreicherung in lipophilen Bestandteilen von Pflanzen entweder nur ungenügend oder gar nicht abzubauen.

Es ist bekannt, dass das Pflanzenschutzmittel Pentachlornitrobenzol in die Abbauprodukte Pentachloranilin, Pentachloranisol und Pentachlorbenzol umgewandelt wird.

Diese Abbauprodukte sind ebenfalls toxisch.

Die genannten hochaktiven toxischen Verbindungen, welche nur ungenügend oder gar nicht abgebaut werden, reichern sich somit im Boden, im Wasser und im menschlichen oder tierischen Körper an, verbunden mit entsprechenden negativen Auswirkungen.

So wird die ganze Nahrungskette, an deren Ende der Mensch ist, vergiftet.

Aus diesen Gründen wurde der Einsatz von bestimmten Wirkstoffen, wie DDT und Lindan, stark eingeschränkt oder verboten.

Der Einsatz von überkritischem Kohlenstoffdioxid (CO₂) ist nur in beschränktem Umfang anwendbar zur Entfernung dieser unerwünschten hochaktiven toxischen Substanzen; siehe beispielsweise EP PS 0 382 116 B1.

Verunreinigtes Wasser kann durch Verwendung von Aktivkohle gereinigt werden.

Dieses Reinigungsverfahren ist sehr teuer und nicht selektiv.

Lipophile Verunreinigungen, insbesondere lipophile Gifte der oben genannten Art, können mit halogenierten Kohlenwasserstoffen, wie Methylenchlorid, Chloroform, Tetrachlorkohlenstoff, oder mit 5 bis 7 Kohlenstoffatome enthaltenden Alkanen, wie Petrolaether, Hexan, entfernt werden.

Diese Stoffe zur Entfernung der lipophilen Gifte der genannten Art sind aber selbst toxisch, umweltschädlich und teilweise hochexplosiv, wodurch deren Anwendung mit grossen Risiken verbunden ist.

Da nicht absehbar war, wann beispielsweise die Nahrungskette mit diesen giftigen Stoffen nicht mehr kontaminiert sein wird, wurden verschiedene nationale und internationale Gesetze und Verordnungen erlassen, in denen Höchstmengen an Giften festgelegt sind.

Es ist ein Ziel der vorliegenden Erfindung, ein Verfahren zur Verfügung zu stellen, mit welchem diese hochaktiven toxischen Verbindungen aus Getränken oder pflanzlichen Zubereitungen nahezu quantitativ und selektiv entfernt werden können.

Dieses Verfahren soll billig sowie einfach und sicher durchführbar sein.

Bei diesem Verfahren sollen keine toxischen und/oder leicht entflammbare Mittel verwendet werden.

Dieses Verfahren soll keine Nachteile gegenüber der Umwelt haben.

Mit diesem Verfahren sollen die in nationalen und internationalen Gesetzen und Verordnungen festgelegten Höchstmengen an Giften zumindest eingehalten und vorzugsweise deutlich unterschritten werden.

Mit dem erfindungsgemässen Verfahren werden die obigen Ziele erreicht.

Das erfindungsgemässe Verfahren zur Entfernung von unerwünschten lipophilen Verunreinigungen und/oder Rückständen, welche in Getränken oder pflanzlichen Zubereitungen enthalten sind,
wobei
die Getränke Wein, Bier, Fruchtsäfte, Tee oder Limonaden sind, und wobei
die pflanzlichen Zubereitungen Aufgüsse, Tinkturen, Fluida, Spissum-Extrakte, Tropflösungen oder Tonika sind, ist dadurch gekennzeichnet, dass man
- in einem ersten Schritt das entsprechende Getränk oder die entsprechende pflanzliche Zubereitung mit einer solchen lipophilen Phase vermischt, dass sich die zu entfernenden Verunreinigungen und/oder Rückstände in dieser lipophilen Phase lösen und sich hierin nahezu quantitativ anreichern,
- in einem zweiten Schritt die lipophile Phase, welche nun die Verunreinigungen und/oder Rückstände enthält, vom entsprechenden Getränk oder von der entsprechenden pflanzlichen Zubereitung abtrennt, und
- in einem dritten Schritt das so gereinigte Getränk oder die so gereinigte pflanzliche Zubereitung gewinnt.

Bevorzugte Ausführungsformen dieses Verfahrens sind in den abhängigen Ansprüchen definiert.

Im folgenden Teil wird das erfindungsgemässe Verfahren näher erläutert. Dabei werden Ausführungsformen, wie sie in den abhängigen Ansprüchen definiert sind, normalerweise nicht wiederholt.

Im zweiten Schritt des erfindungsgemässen Verfahrens erfolgt eine Separation von zwei Phasen.

Dabei werden entweder zwei flüssige Phasen oder eine flüssige Phase und eine feste Phase voneinander getrennt.

Wenn zwei flüssige Phasen voneinander getrennt werden, dann wird dies vorzugsweise mittels Membrantechnologie bewerkstelligt.

Dabei sind röhrenförmige Membranen, sogenannte "cross-flow"-Membranen, bevorzugt.

Dabei wird die verwendete Membran vorzugsweise vorgängig konditioniert.

Wenn gewünscht wird, dass die hydrophile Phase als Filtrat und die lipophile Phase als Retentat erhalten werden, dann wird die Membran mit einem hydrophilen Lösungsmittel, insbesondere Wasser, oder mit einem hydrophilen Lösungsmittelgemisch, beispielsweise ein Gemisch aus 85 Vol.-% Wasser und 15 Vol.-% Ethanol, während einigen Minuten, beispielsweise 5 bis 20 Minuten, insbesondere 10 Minuten, bei Raumtemperatur konditioniert.

Es ist prinzipiell auch möglich, die Membran mit der hydrophilen Phase selbst zu konditionieren.

Wenn gewünscht wird, dass die lipophile Phase als Filtrat und die hydrophile Phase als Retentat erhalten werden, dann wird die Membran, in Analogie zu obigen Ausführungen, mit einem lipophilen Lösungsmittel oder Lösungsmittelgemisch oder mit der lipophilen Phase selbst konditioniert.

Es ist bevorzugt, die hydrophile Phase als Filtrat zu gewinnen.

Nach dieser Konditionierung der Membran wird das zu separierende Gemisch, beispielsweise 9 Teile hydrophile Zubereitung und 1 Teil lipophile Phase, der oben genannten kontinuierlichen Membran-Separation so lange unterworfen, bis die gewünschte Menge der gereinigten hydrophilen Zubereitung als Filtrat erhalten wird.

Wenn eine flüssige Phase und eine feste Phase voneinander getrennt werden, dann wird dies mittels herkömmlicher Technologie bewerkstelligt.

In diesem Falle wird Kakaobutter als lipophile Phase bevorzugt.

Dabei wird in die zu reinigende hydrophile Zubereitung, welche vorzugsweise eine Temperatur von etwa 50°C hat, Kakaobutter im geschmolzenen Zustand eingerührt.

Dieses Gemisch wird während etwa einer Stunde bei einer Temperatur von etwa 50°C gerührt.

Danach lässt man das Gemisch auf Raumtemperatur oder auf eine Temperatur im Bereich von 5°C bis 10°C, insbesondere 8°C, abkühlen.

Bei diesen Temperaturen wird die Kakaobutter fest und kann von der gereinigten hydrophilen Zubereitung abgetrennt werden.

Im erfindungsgemässen Verfahren sind die hydrophile Phase und die lipophile Phase während des Extraktionsschrittes vorzugsweise je in flüssigem Aggregatzustand.

Die nachfolgenden Beispiele illustrieren die vorliegende Erfindung.

### Beispiel 1

400 g Extr. Ginseng e. rad. spir. spiss. wurden mit 800 g destilliertem Wasser versetzt und unter Rühren auf eine Temperatur von 50°C gebracht.

Nach Erreichen der Temperatur wurden 40 g Kakaobutter in geschmolzenem Zustand eingerührt, und diese Mischung wurde während 1 Stunde bei einer Temperatur von 50°C gerührt.

Danach wurde die Mischung für 2 Tage bei 8°C aufbewahrt.

Dann wurde die sich an der Oberfläche des Gemisches befindliche feste Masse, welche Kakaobutter und die Verunreinigungen enthielt, abgehoben und verworfen.

Der Rückstand, also die flüssige Extraktlösung, wurde über ein Faltenfilter filtriert.

Das erhaltene Filtrat wurde unter Vakuum bei einer Temperatur von maximal 50°C auf das Ausgangsgewicht von 400 g eingeengt.

Die Pestizidwerte des so erhaltenen Produktes sind in der Tabelle 1 erwähnt.

### Beispiel 2

1000 g Extrakt Ginseng e. rad. spir. spiss. wurden mit 4000 g destilliertem Wasser versetzt und unter Rühren auf eine Temperatur von 50°C gebracht.

Nach Erreichen der Temperatur wurden 100 g Kakaobutter in geschmolzenem Zustand eingerührt, und diese Mischung wurde während 1 Stunde bei einer Temperatur von 50°C gerührt.

Die Mischung wurde dann auf 30°C abgekühlt und mittels Cross-Flow auf einer Ultrafiltrationsanlage wähend 2 Stunden filtriert.

Die Membrane (Polypropylen-Röhrenmodul) der Cross-Flow-Anlage wurde vorher während 10 Minuten mit destilliertem Wasser, welches eine Temperatur von 30 bis 42°C hatte, konditioniert.

Das erhaltene Filtrat wurde bei einer Temperatur von maximal 50°C auf das Ausgangsgewicht von 1000 g eingeengt.

Die Pestizidwerte des so erhaltenen Produktes sind in der Tabelle 1 erwähnt.

### Beispiel 3

500 g Extrakt Ginseng e. rad. spir. spiss. wurden mit 2000 g destilliertem Wasser versetzt und 15 Minuten bei einer Temperatur von 50°C gerührt.

Dann wurden 50 g Miglyol 812 zugegeben, und es wurde eine weitere Stunde bei einer Temperatur von 50°C gerührt.

Dieses Gemisch wurde über Nacht bei Raumtemperatur stehen gelassen.

Dann wurde die Mischung mittels Cross-Flow auf einer Ultrafiltrationsanlage während 1 Stunde filtriert.

Die Membrane (Polypropylen-Röhrenmodul) der Cross-Flow-Anlage wurde vorher während 10 Minuten mit destilliertem Wasser bei Raumtemperatur konditioniert.

Das erhaltene Filtrat wurde bei einer Temperatur von maximal 50°C auf das Ausgangsgewicht von 500 g eingeengt.

Die Pestizidwerte des so erhaltenen Produktes sind in der Tabelle 1 erwähnt.

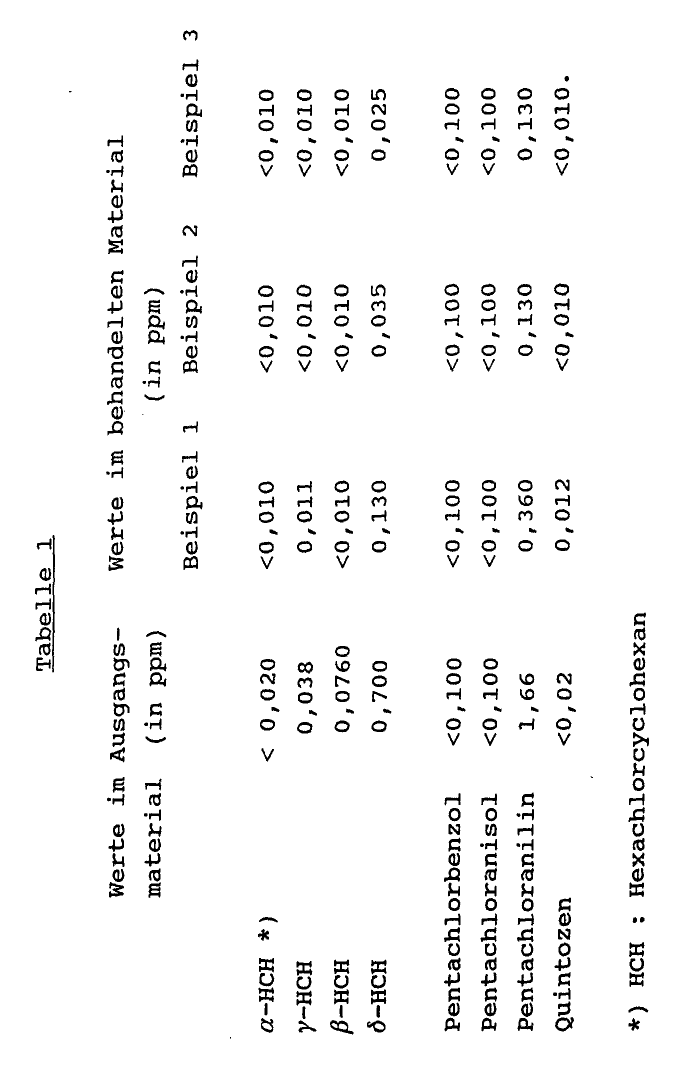

## Patentansprüche

1. Verfahren zur Entfernung von unerwünschten lipophilen Verunreinigungen und/oder Rückständen, welche in Getränken oder pflanzlichen Zubereitungen enthalten sind, wobei
die Getränke Wein, Bier, Fruchtsäfte, Tee oder Limonaden sind, und wobei
die pflanzlichen Zubereitungen Aufgüsse, Tinkturen, Fluida, Spissum-Extrakte, Tropflösungen oder Tonika sind,
**dadurch gekennzeichnet, dass** man
- in einem ersten Schritt das entsprechende Getränk oder die entsprechende pflanzliche Zubereitung mit einer solchen lipophilen Phase vermischt, dass sich die zu entfernenden Verunreinigungen und/oder Rückstände in dieser lipophilen Phase lösen und sich hierin nahezu quantitativ anreichern,
- in einem zweiten Schritt die lipophile Phase, welche nun die Verunreinigungen und/oder Rückstände enthält, vom entsprechenden Getränk oder von der entsprechenden pflanzlichen Zubereitung abtrennt, und
- in einem dritten Schritt das so gereinigte Getränk oder die so gereinigte pflanzliche Zubereitung gewinnt.

2. Verfahren nach Anspruch 1, **dadurch gekennzeichnet, dass** die unerwünschten lipophilen Verunreinigungen und/oder Rückstände Pestizide, Pflanzenschutzmittel, Schädlingsbekämpfungsmittel, insbesondere Fungizide, Insektizide, Akarizide, Nematizide, Herbizide, oder Umweltgifte, wie polyhalogenierte, insbesondere polychlorierte, Biphenyle, Dioxine, oder organische Lösungsmittel, wie Benzol, Chloroform, Tetrachlorkohlenstoff, oder Syntheserückstände, wie Alkylhalogenide, halogenierte Aromaten und Heteroaromaten, wie Chlorpyridine, sind, einschliesslich beliebige Gemische davon.

3. Verfahren nach einem der Ansprüche 1 bis 2, **dadurch gekennzeichnet, dass** die unerwünschten lipophilen Verunreinigungen und/oder Rückstände ausgewählt sind aus der Gruppe, bestehend aus
α - Hexachlorcyclohexan,
β - Hexachlorcyclohexan,
γ - Hexachlorcyclohexan, auch Lindan genannt,
δ - Hexachlorcyclohexan,
Pentachlornitrobenzol, auch Quintozen genannt, und dessen Abbauprodukte, wie Pentachloranilin, Pentachloranisol, Pentachlorbenzol,
Dichlor-diphenyl-trichlor-ethan, auch DDT genannt, und dessen Abbauprodukte, wie Dichlor-diphenyldichlor-aethylen, auch DDE genannt,
Endosulfan, auch Thiodan genannt,
Pyrethrum, einschliesslich dessen Synergisten,
Piperonylbutoxid,
Hexachlorbenzol,
Aldrin,
Dieldrin,
Heptachlor,
Methoxychlor.

4. Verfahren nach einem der Ansprüche 1 bis 3, **dadurch gekennzeichnet, dass** die pflanzlichen Zubereitungen Teil- oder Vollextrakte aus Heil- und/oder Gewürzpflanzen oder Teilen davon sind, insbesondere ausgewählt aus der Gruppe, bestehend aus
Abelmoschus moschatus L (Semen)
Acorus calamus (Rhizom)
Aesculus hippocastanum L. (Semen)
Allium-Arten (z.B. A. cepa L., A. ursinum L., A. sativum L.:Bulbus)
Alpinia officinarum Hance (Rhizom)
Anethum graveolens L. (Fructus)
Angelica archangelica L., verschiedene Subspec. (Rhizoma)
Angelica dahurica (Radix)
Angelica formosana (Radix)
Anthemis nobilis L. (Chamomilla romana, Herba)
Apium graveolens L. (Fructus)
Arctium major Gaertn. (Radix)
Arctostaphylos uva-ursi Spreng. (Folium)
Arnica montana L. (Flos)
Artemisia absinthium L. (Herba)
Artemisia dracunculus L. (Herba)
Asparagus offic. (Herba, Rhizoma, Radix)
Atropa belladonna L. (Folium)
Berberis vulgaris L. (Cortex, Radix)
Betula-Arten (Folium)
Brassica nigra (L.) Koch (Semen)
Camellia sinensis (Folia)
Carum carvi L. (Fructus)
Cetraria islandica (L.) Ach.
Chrysanthemum vulgare Asch. (Herba)
Cinnamomum-Arten, (Cortex)
Citrus-Arten (Folium, Flavedo, Fruct.)
Copaifera reticulata Ducke (Balsam)
Coriandrum sativum L. (Fructus)
Cucurbita pepo L. (Semen)
Cuminum cyminum L. (Fructus)
Curcuma-Arten (Rhizoma)
Cusparia officinalis (Willd.) Eng. (Cortex)
Dipterocarpus turbinatus Gaertn. (Balsamum)
Drosera Arten (D. rotundifolia L., D.ramentacea Burch; Herba)
Echinacea angustifolia D.C. (Radix)
Echinacea purpurea (L.) Moench (Radix)
Elettaria cardamonum (L.) White et Mathon (Fructus)
Equisetum arvense L. (Herba)
Eucalyptus globulus Labill. (Folium)
Fagopyrum vulgare Hill. (Herba)
Foeniculum vulgare Miller (Fructus)
Fumaria offic. (Herba)
Gaultheria procumbens L. (Folium)
Ginkgo biloba L. (Folium)
Hamamelis virginiana L. (Cortex, Folium)
Hedeoma pulegioides (L.) Pers. (Herba)
Herniaria glabra L. (Herba)
Humulus lupulus L. (Flos, Glandulae)
Hypericum perforatum L. (Herba)
Hysopus officinalis L. (Herba)
Ilex paraguariensis St. Hil. (Folium mate)
Illicium verum Hook. f. (Fructus)
Iluna helenium L. (Rhizoma)
Iris pallida Lam. (Rhizoma)
Jasminum grandiflorum L. (Flos)
Laurus nobilis L. (Folium, Fructus)
Lavendula officinalis, weitere Arten (Flos)
Lawsonia inermis L. (Folium)
Levisticum officinale Koch (Radix)
Melaleuca: div. Varietäten (Folium)
Matricaria chamomilla L. (Flos)
Melilotus officinalis (L.) Lam. em. Thuill. (Herba)
Melissa officinalis L. (Herba)
Mentha-Arten und ihre Varietäten (Folium)
Myristica fragrans Houttuyn (Arillus, Semen)
Myrtus communis L. (Folium)
Ocimum basilicum L. (Herba)
Ocotea sassafras (Cortex)
Oenanthe aquatica (L.) Poir (Fructus)
Olea europaea (Folia)
Olibanum (Resinum)
Ononis spinosa L. (Radix)
Origanum-Arten (Herba)
Orthosiphon stamineus Benth. (Herba)
Panax ginseng Meyer (Radix)
Petroselinum crispum (Mill.) Nym. (Fructus, Herba)
Phaseolus vulgaris L. (Fructus sine Semine)
Pimenta dioica (L.) Merill (Fructus)
Pimpinella anisum L. (Semen)
Piper angustifolium Ruiz. et Pavon. (Folium)
Piper methysticum Forster (Radix)
Pogostemon patchouli Pell. (Folium)
Prunus laurocerasus L. (Folium)
Rhus aromatica Ait. (Cortex)
Rosmarinus officinalis L. und ihre Species (Folium)
Rubia tinctorum L. (Radix)
Rubus fructicosus L. (Folium)
Ruta graveolens L. (Herba)
Sabal serulata Benth et Hook (Fructus)
Salix alba L. (Cortex) und alle Arten
Salvia-Arten (Folium)
Santalum album L. (Lignum)
Sarothamnus scoparius (L.) Wimmer (Herba)
Sassafras albidum (Nutt.) Nees (Lignum)
Satureja hortensis L. (Herba)
Scopolia carniolica Jacq. (Radix)
Solidago serotina Ait. (Herba)
Solidago virgaurea L. (Herba)
Syzygium aromaticum Merr. et Perry (Flores, Folium)
Taraxacum officinale Web. (Herba und Radix)
Thymus serpyllum L. (Herba)
Thymus vulgaris L. Herba
Tilia cordata Mill. und T. platyphyllos Scop. (Flos)
Urtica dioica L. (Folium, Radix)
Valeriana officinalis und ihre Varietäten (Radix)
Vitis vinifera (Folia)
Zingiberis officinale Roscoe (Rhizoma).

5. Verfahren nach einem der Ansprüche 1 bis 4, **dadurch gekennzeichnet, dass** die pflanzlichen Zubereitungen Teil- oder Vollextrakte aus Alkaloide und/oder Flavonoide und/oder Saponine und/oder Bitterstoffe und/oder Terpene enthaltenden Pflanzen oder Teilen davon sind, insbesondere ausgewählt aus der Gruppe, bestehend aus
Betulae (Folia)
Boldo (Folia)
Camelliae (Folia)
Chelidonii (Herba)
Chinae (Cortex)
Chrysanthemi (Herba)
Crataegi (Folia c. Floribus)
Cynarae (Folia)
Gentianae (Radix)
Ginkgo (Folia)
Ginseng (Radix)
Hederae helic. (Herba)
Hippocastani (Semen)
Liquiritiae (Radix)
Orthosiphonis (Folia)
Passiflorae (Herba)
Rauwolfiae (Radix)
Salicis (Cortex)
Solidaginis (Herba)
Tiliae (Flores)
Vitis vinifera (Folia, Fructus).

6. Verfahren nach einem der Ansprüche 1 bis 5, **dadurch gekennzeichnet, dass** die lipophile Phase tierischen, pflanzlichen, mineralischen oder synthetischen Ursprungs ist, und insbesondere nicht toxisch, nicht leicht entzündlich, nicht explosiv und nicht flüchtig ist, und vorzugsweise ausgewählt ist aus
- Fetten, wie Kakaobutter, Kokosfett;
- Oelen, wie Neutralöle, Sonnenblumenöl, fraktioniertes Kokosnussöl, wie Miglyol;
- Wachsen, wie Stearine, Yoyobaöl, Bienenwachs, Walrat, Carnaubawachs;
- Paraffinen, einschliesslich Vaseline;
- Lipoiden; und
- Sterinen,
wobei alle diese Verbindungen, einzeln oder als Gemisch, vorzugsweise die Erfordernisse/Definitionen im Deutschen Arzneibuch, DAB, oder in der Britischen Pharmacopoe, BP, oder gemäss dem Food Chemical Codex, FCC, in den Vereinigten Staaten von Amerika erfüllen, respektive diesen Erfordernissen/Definitionen entsprechen müssen.

7. Verfahren nach einem der Ansprüche 1 bis 6, **dadurch gekennzeichnet, dass** im ersten Schritt die Vermischung der Komponenten bei einer solchen Temperatur erfolgt, welche zwischen dem Gefrierpunkt und dem Siedepunkt des jeweiligen Gemisches liegt, wobei eine Temperatur im Bereich von Raumtemperatur bis 70°C bevorzugt ist.

8. Verfahren nach einem der Ansprüche 1 bis 7, **dadurch gekennzeichnet, dass** man im ersten Schritt die Komponenten während etwa 1 Stunde miteinander vermischt, insbesondere durch Schütteln oder Rühren.

9. Verfahren nach einem der Ansprüche 1 bis 8, **dadurch gekennzeichnet, dass** im zweiten Schritt die Abtrennung entweder mittels einer Phasentrennung von 2 flüssigen Phasen oder mittels einer Phasentrennung einer flüssigen und einer festen Phase erfolgt.

10. Verfahren nach einem der Ansprüche 1 bis 9, **dadurch gekennzeichnet, dass** im zweiten Schritt die Abtrennung von 2 flüssigen Phasen mittels Membran-Separation erfolgt, wobei Membranen mit Porenöffnungen im Bereich von 0,001 bis 1,0 Mikrometer, insbesondere von 0,1 bis 0,3 Mikrometer, bevorzugt sind, insbesondere Glas-, Metall-, Keramik- und Kunststoffmembranen, beispielsweise aus Polypropylen oder Teflon.

11. Verfahren nach einem der Ansprüche 1 bis 10, **dadurch gekennzeichnet, dass** man die im zweiten Schritt abgetrennte lipophile Phase, welche die Verunreinigungen und/oder Rückstände enthält, einer Wasserdampfdestillation unterwirft, das erhaltene Destillat welches lipophile, wasserdampfflüchtige Geruchsstoffe und/oder Geschmacksstoffe enthält, als solches oder nach vorheriger Entfernung des Wassers mit dem am Ende des dritten Schrittes erhaltenen gereinigten Getränk oder mit der am Ende des dritten Schrittes erhaltenen gereinigten pflanzlichen Zubereitung kombiniert.

12. Verfahren nach einem der Ansprüche 1 bis 11, **dadurch gekennzeichnet, dass** man zu pflanzlichen Zubereitungen, welche Alkaloide enthalten, vor der Durchführung des ersten Schrittes wenigstens eine physiologisch unbedenkliche Säure, beispielsweise Ascorbinsäure, Zitronensäure, Essigsäure, in einer solchen Menge zugibt, dass ein solcher pH-Wert erhalten wird, dass die Alkaloide als Salze vorliegen.

## Claims

1. A process for the removal of undesired lipophilic contaminations and/or residues, which are contained in beverages or in vegetable preparations, whereby
the beverages are wine, beer, fruit-juces, tea or lemonades, and whereby
the vegetable preparations are infusions, tinctures, fluids, spissum extracts, dropping solutions or tonics,
**characterized in that**
- in a first step the corresponding beverage or the corresponding vegetable preparation is mixed with such a lipophilic phase, that the contaminations and/or residues to be removed are dissolved in this lipophilic phase and are concentrated herein nearly quantitatively,
- in a second step the liphophilic phase, which contains now the contaminations and/or residues, is separated from the corresponding beverage or from the corresponding vegetable preparation, and
- in a third step the so purified beverage or the so purified vegetable preparation is obtained.

2. The process according to claim 1, **characterized in that** the undesired lipophilic contaminations and/or residues are pesticides, plant protective products, pest control agents, especially fungicides, insecticides, acaricides, nematicides, herbicides, or environmental poisons, such as polyhalogenated, especially polychlorinated, biphenyls, dioxines, or organic solvents, such as benzene, chloroform, carbon tetrachloride, or synthesis residues, such as alkylhalides, halogenated aromatic substances and heteroatomic aromatic substances, such as chloropyridines, including any mixtures thereof.

3. The process according to one of claims 1 to 2, **characterized in that** the undesired lipophilic contaminations and/or residues are selected from the group, consisting of
α - hexachloro cyclohexane,
β - hexachloro cyclohexane,
γ - hexachloro cyclohexane, also named lindan,
δ - hexachloro cyclohexane,
pentachloro nitrobenzene, also named quintozen, and its degradation products, such as pentachloro aniline, pentachloro anisol, pentachloro benzene,
dichloro-diphenyl-trichloro-ethane, also named DDT, and its degradation products, such as dichloro-diphenyl-dichloro-ethylene, also named DDE ,
endosulfan, also named thiodan,
pyrethrum, including its synergists,
piperonylbutoxide,
hexachloro benzene,
aldrin,
dieldrin,
heptachlor,
methoxychlor.

4. The process according to one of claims 1 to 3, **characterized in that** the vegetable preparations are partial or complete extracts from medical and/or spice plants or parts thereof, especially selected from the group, consisting of
Abelmoschus moschatus L (Semen)
Acorus calamus (Rhizom)
Aesculus hippocastanum L. (Semen)
Allium-species (e.g. A. cepa L., A. ursinum L., A. sativum L.:Bulbus)
Alpinia officinarum Hance (Rhizom)
Anethum graveolens L. (Fructus)
Angelica archangelica L., various subspec. (Rhizoma)
Angelica dahurica (Radix)
Angelica formosana (Radix)
Anthemis nobilis L. (Chamomilla romana, Herba)
Apium graveolens L. (Fructus)
Arctium major Gaertn. (Radix)
Arctostaphylos uva-ursi Spreng. (Folium)
Arnica montana L. (Flos)
Artemisia absinthium L. (Herba)
Artemisia dracunculus L. (Herba)
Asparagus offic. (Herba, Rhizoma, Radix)
Atropa belladonna L. (Folium)
Berberis vulgaris L. (Cortex, Radix)
Betula-species (Folium)
Brassica nigra (L.) Koch (Semen)
Camellia sinensis (Folia)
Carum carvi L. (Fructus)
Cetraria islandica (L.) Ach.
Chrysanthemum vulgare Asch. (Herba)
Cinnamomum-species (Cortex)
Citrus-species (Folium, Flavedo, Fruct.)
Copaifera reticulata Ducke (Balsam)
Coriandrum sativum L. (Fructus)
Cucurbita pepo L. (Semen)
Cuminum cyminum L. (Fructus)
Curcuma-species (Rhizoma)
Cusparia officinalis (Willd.) Eng. (Cortex) Dipterocarpus turbinatus Gaertn. (Balsamum)
Drosera species (D. rotundifolia L., D.ramentacea Burch; Herba)
Echinacea angustifolia D.C. (Radix)
Echinacea purpurea (L.) Moench (Radix)
Elettaria cardamonum (L.) White et Mathon (Fructus)
Equisetum arvense L. (Herba)
Eucalyptus globulus Labill. (Folium)
Fagopyrum vulgare Hill. (Herba)
Foeniculum vulgare Miller (Fructus)
Fumaria offic. (Herba)
Gaultheria procumbens L. (Folium)
Ginkgo biloba L. (Folium)
Hamamelis virginiana L. (Cortex, Folium)
Hedeoma pulegioides (L.) Pers. (Herba)
Herniaria glabra L. (Herba)
Humulus lupulus L. (Flos, Glandulae)
Hypericum perforatum L. (Herba)
Hysopus officinalis L. (Herba)
Ilex paraguariensis St. Hil. (Folium mate)
Illicium verum Hook. f. (Fructus)
Iluna helenium L. (Rhizoma)
Iris pallida Lam. (Rhizoma)
Jasminum grandiflorum L. (Flos)
Laurus nobilis L. (Folium, Fructus)
Lavendula officinalis, further species (Flos)
Lawsonia inermis L. (Folium)
Levisticum officinale Koch (Radix)
Melaleuca: various varieties (Folium)
Matricaria chamomilla L. (Flos)
Melilotus officinalis (L.) Lam. em. Thuill. (Herba)
Melissa officinalis L. (Herba)
Mentha-species and its varieties (Folium)
Myristica fragrans Houttuyn (Arillus, Semen)
Myrtus communis L. (Folium)
Ocimum basilicum L. (Herba)
Ocotea sassafras (Cortex)
Oenanthe aquatica (L.) Poir (Fructus)
Olea europaea (Folia)
Olibanum (Resinum)
Ononis spinosa L. (Radix)
Origanum-species (Herba)
Orthosiphon stamineus Benth. (Herba)
Panax ginseng Meyer (Radix)
Petroselinum crispum (Mill.) Nym. (Fructus, Herba)
Phaseolus vulgaris L. (Fructus sine Semine)
Pimenta dioica (L.) Merill (Fructus)
Pimpinella anisum L. (Semen)
Piper angustifolium Ruiz. et Pavon. (Folium)
Piper methysticum Forster (Radix)
Pogostemon patchouli Pell. (Folium)
Prunus laurocerasus L. (Folium)
Rhus aromatica Ait. (Cortex)
Rosmarinus officinalis L. and its species (Folium)
Rubia tinctorum L. (Radix)
Rubus fructicosus L. (Folium)
Ruta graveolens L. (Herba)
Sabal serulata Benth et Hook (Fructus)
Salix alba L. (Cortex) and all species
Salvia-species (Folium)
Santalum album L. (Lignum)
Sarothamnus scoparius (L.) Wimmer (Herba)
Sassafras albidum (Nutt.) Nees (Lignum)
Satureja hortensis L. (Herba)
Scopolia carniolica Jacq. (Radix)
Solidago serotina Ait. (Herba)
Solidago virgaurea L. (Herba)
Syzygium aromaticum Merr. et Perry (Flores, Folium)
Taraxacum officinale Web. (Herba and Radix)
Thymus serpyllum L. (Herba)
Thymus vulgaris L. Herba
Tilia cordata Mill. and T. platyphyllos Scop. (Flos)
Urtica dioica L. (Folium, Radix)
Valeriana officinalis and its varieties (Radix)
Vitis vinifera (Folia)
Zingiberis officinale Roscoe (Rhizoma).

5. The process according to one of claims 1 to 4, **characterized in that** the vegetable preparations are partial or complete extracts from alkaloids and/or flavonoides and/or saponines and/or bitterings and/or terpenes containing plants or parts thereof, especially selected from the group, consisting of
Betulae (Folia)
Boldo (Folia)
Camelliae (Folia)
Chelidonii (Herba)
Chinae (Cortex)
Chrysanthemi (Herba)
Crataegi (Folia c. Floribus)
Cynarae (Folia)
Gentianae (Radix)
Ginkgo (Folia)
Ginseng (Radix)
Hederae helic. (Herba)
Hippocastani (Semen)
Liquiritiae (Radix)
Orthosiphonis (Folia)
Passiflorae (Herba)
Rauwolfiae (Radix)
Salicis (Cortex)
Solidaginis (Herba)
Tiliae (Flores)
Vitis vinifera (Folia, Fructus).

6. The process according to one of claims 1 to 5, **characterized in that** the liphophilic phase is of animal, vegetable, mineralic or synthetic origin, and is especially not toxic, not easily inflammable, not explosive and not volatile, and is preferably selected from
- fats, such as cocoa butter, coconut fat;
- oils, such as neutral oils, sunflower oil, fractionated coconut oil, such as miglyol;
- waxes, such as stearins, yoyoba oil, beeswax, spermaceti, carnauba wax;
- paraffins, including vaseline;
- lipoids; and
- sterols,
whereby all these compounds, as a simple compound or as a mixture, preferably fullfill the requirements/definitions in the "Deutsches Arzneibuch, DAB", or in the British Pharmacopoe, BP, or according to the Food Chemical Codex, FCC, in the United States of America, or must correspond to these requirements/definitions, respectively.

7. The process according to one of claims 1 to 6, **characterized in that** in the first step the mixing of the components is realized at such a temperature, which lies between the freezing point and the boiling point of the respective mixture, whereby a temperature in the range from room temperature to 70°C is preferred.

8. The process according to one of claims 1 to 7, **characterized in that** in the first step the components are mixed together during about 1 hour, especially by means of shaking or stirring.

9. The process according to one of claims 1 to 8, **characterized in that** in the second step the separation is realized either by means of a phase separation of 2 liquid phases or by means of a phase separation of a liquid phase and a solid phase.

10. The process according to one of claims 1 to 9, **characterized in that** in the second step the separation of 2 liquid phases is realized by means of membrane-separation, whereby membranes with pore sizes in the range from 0.001 to 1.0 micrometer, especially from 0.1 to 0.3 micrometer, are preferred, especially glass-, metal-, ceramic- and synthetic membranes, for example made of polypropylene or teflon.

11. The process according to one of claims 1 to 10, **characterized in that** the in the second step separated lipophilic phase, which contains the contaminations and/or residues, is subjected to a water vapor distillation, the obtained distillate, which contains the lipophilic, volatile-in-steam smell components and/or taste components, is combined as such or after previous removal of the water with the at the end of the third step obtained purified beverage or with the at the end of the third step obtained purified vegetable preparation.

12. The process according to one of claims 1 to 11, **characterized in that** to vegetable preparations, which contain alkaloids, are added before the realization of the first step at least one physiologically acceptable acid, for example ascorbic acid, citric acid, acetic acid, in such an amount that such a pH-value is obtained, that the alkaloids are present as salts.

## Revendications

1. Procédé pour éliminer des impuretés et/ou des résidus lipophiles indésirables qui sont contenus dans des boissons ou dans des préparations végétales, dans lequel les boissons sont le vin, la bière, les jus de fruits, le thé ou les limonades et dans lequel les préparations végétales sont des infusions, des teintures, des fluides, des extraits concentrés, des solutions d'écoulement en goutte ou des boissons toniques
**caractérisé en ce que**
- dans une première étape, on mélange la boisson correspondante ou la préparation végétale correspondante avec une phase lipophile de telle sorte que les impuretés et/ou les résidus à éliminer se dissolvent dans cette phase lipophile et s'y enrichissent de manière pratiquement quantitative,
- dans une deuxième étape, on sépare la phase lipophile qui contient maintenant les impuretés et/ou les résidus, de la boisson correspondante ou de la préparation végétale correspondante, et
- dans une troisième étape, on obtient la boisson ainsi purifiée ou la préparation végétale ainsi purifiée.

2. Procédé selon la revendication 1, **caractérisé en ce que** les impuretés et/ou les résidus lipophiles indésirables sont des pesticides, des agents de protection des plantes, des agents de lutte contre les parasites, en particulier des fongicides, des insecticides, des acaricides, des nématicides, des herbicides ou des polluants tels que des biphényles polyhalogénés, en particulier polychlorés, des dioxines ou encore des solvants organiques tels que le benzène, le chloroforme, le tétrachlorure de carbone, ou bien des résidus de synthèse tels que des halogénures d'alkyle, des composés aromatiques et hétéroaromatiques halogénés tels que les chloropyridines, y compris n'importe quels mélanges de ces impuretés et/ou de ces résidus.

3. Procédé selon l'une quelconque des revendications 1 à 2, **caractérisé en ce que** les impuretés et/ou les résidus lipophiles indésirables sont sélectionnés parmi le groupe constitué par
l'α-hexachlorocyclohexane,
le β-hexachlorocyclohexane,
le γ-hexachlorocyclohexane, également appelé Lindane®,
le δ-hexachlorocyclohexane,
le pentachloronitrobenzène également appelé quintozène et ses produits de décomposition tels que la pentachloro-aniline, le pentachloroanisole, le pentachlorobenzène,
le dichloro-diphényl-trichloréthane, également désigné par l'abréviation DDT et ses produits de décomposition tels que le dichloro-diphényldichloréthylène également désigné par l'abréviation DDE,
l'endosulfane également appelé Thiodan,
le pyrèthre, y compris ses agents synergiques,
le butoxyde de pipéronyle,
l'hexachlorobenzène,
l'aldrine,
la dieldrine,
l'Heptachlor,
le méthoxychlore.

4. Procédé selon l'une quelconque des revendications 1 à 3, **caractérisé en ce que** les préparations végétales sont des extraits partiels ou complets de plantes médicinales et/ou de condiments ou encore des parties de ces derniers, en particulier choisis parmi le groupe constitué par
Abelmoschus moschatus L (Semen)
Acorus calamus (Rhizoma)
Aesculus hippocastanum L. (Semen)
les espèces Allium (par exemple, A. cepa L., A. ursinum L., A. sativum L.: Bulbus)
Alpinia officinarum Hance (Rhizoma)
Anethum graveolens L. (Fructus)
Angelica archangelica L., diverses sous-espèces (Rhizoma)
Angelica dahurica (Radix)
Angelica formosana (Radix)
Anthemis nobilis L. (Chamomilla romana, Herba)
Apium graveolens L. (Fructus)
Arctium major Gaertn. (Radix)
Arctostaphylos uva-ursi Spreng. (Folium)
Arnica montana L. (Flos)
Artemisia absinthium L. (Herba)
Artemisia dracunculus L. (Herba)
Asparagus offic. (Herba, Rhizoma, Radix)
Atropa belladona L. (Folium)
Berberis vulgaris L. (Cortex, Radix)
les espèces Betula (Folium)
Brassica nigra (L.) Koch (Semen)
Camellia sinensis (Folia)
Carum carvi L. (Fructus)
Cetraria islandica (L.) Ach.
Chrysanthemum vulgare Asch. (Herba)
les espèces Cinnamomum, (Cortex)
les espèces Citrus (Folium, Flavedo, Fruct.)
Copaifera reticulata Ducke (Balsamum)
Coriandrum sativum L. (Fructus)
Cucurbita pepo L. (Semen)
Cuminum cyminum L. (Fructus)
les espèces Curcuma (Rhizoma)
Cusparia officinalis (Willd.) Eng. (Cortex)
Dipterocarpus turbinatus Gaertn. (Balsamum)
les espèces Drosera (D. rotundifolia L., D. ramentacea Burch; Herba)
Echinacea angustifolia D.C. (Radix)
Echinacea purpurea (L.) Moench (Radix)
Elettaria cardamonum (L.) White et Mathon (Fructus)
Equisetum arvense L. (Herba)
Eucalyptus globulus Labill. (Folium)
Fagopyrum vulgare Hill. (Herba)
Foeniculum vulgare Miller (Fructus)
Fumaria offic. (Herba)
Gaultheria procumbens L. (Folium)
Ginkgo biloba L. (Folium)
Hamamelis virginiana L. (Cortex, Folium)
Hedeoma pulegioides (L.) Pers. (Herba)
Herniaria glabra L. (Herba)
Humulus lupulus L. (Flos, Glandulae)
Hypericum perforatum L. (Herba)
Hysopus officinalis L. (Herba)
Ilex paraguariensis St. Hil. (Folium mate)
Illicium verum Hook. f. (Fructus)
Iluna helenium L. (Rhizoma)
Iris pallida Lam. (Rhizoma)
Jasminum grandiflorum L. (Flos)
Laurus nobilis L. (Folium, Fructus)
Lavendula officinalis, d'autres espèces (Flos)
Lawsonia inermis L. (Folium)
Levisticum officinale Koch (Radix)
Melaleuca: différentes variétés (Folium)
Matricaria chamomilla L. (Flos)
Melilotus officinalis (L.) Lam. em. Thuill. (Herba)
Melissa officinalis L. (Herba)
les espèces Mentha et leurs variétés (Folium)
Myristica fragrans Houttuyn (Arillus, Semen)
Myrtus communis L. (Folium)
Ocimum basilicum L. (Herba)
Ocotea sassafras (Cortex)
Oenanthe aquatica (L.) Poir (Fructus)
Olea europaea (Folia)
Olibanum (Resinum)
Ononis spinosa L. (Radix)
les espèces Origanum (Herba)
Orthosiphon stamineus Benth. (Herba)
Panax ginseng Meyer (Radix)
Petroselinum crispum (Mill.) Nym. (Fructus, Herba)
Phaseolus vulgaris L. (Fructus sine Semine)
Pimenta dioica (L.) Merill (Fructus)
Pimpinella anisum L. (Semen)
Piper angustifolium Ruiz. et Pavon (Folium)
Piper methysticum Forster (Radix)
Pogostemon patchouli Pell. (Folium)
Prunus laurocerasus L. (Folium)
Rhus aromatica Ait. (Cortex)
Rosmarinus officinalis L. et ses espèces (Folium)
Rubia tinctorum L. (Radix)
Rubus fructicosus L. (Folium)
Ruta graveolens L. (Herba)
Sabal serulata Benth et Hook (Fructus)
Salix alba L. (Cortex) et toutes les espèces
les espèces Salvia (Folium)
Santalum album L. (Lignum)
Sarothamnus scoparius (L.) Wimmer (Herba)
Sassafras albidum (Nutt.) Nees (Lignum)
Satureja hortensis L. (Herba)
Scopolia carniolica Jacq. (Radix)
Solidago serotina Ait. (Herba)
Solidago virgaurea L. (Herba)
Syzygium aromaticum Merr. et Perry (Flores, Folium)
Taraxacum officinale Web. (Herba et Radix)
Thymus serpyllum L. (Herba)
Thymus vulgaris L. (Herba)
Tilia cordata Mill. et T. platyphyllos Scop. (Flos)
Urtica dioica L. (Folium, Radix)
Valeriana officinalis et ses variétés (Radix)
Vitis vinifera (Folia)
Zingiberis officinale Roscoe (Rhizoma).

5. Procédé selon l'une quelconque des revendications 1 à 4, **caractérisé en ce que** les préparations végétales sont des extraits partiels ou complets de plantes ou de parties de ces dernières, contenant des alcaloïdes et/ou des flavonoïdes et/ou des saponines et/ou des substances amères et/ou des terpènes, en particulier choisis parmi le groupe constitué par
Betulae (Folia)
Boldo (Folia)
Camelliae (Folia)
Chelidonii (Herba)
Chinae (Cortex)
Chrysanthemi (Herba)
Crataegi (Folia c. Floribus)
Cynarae (Folia)
Gentianae (Radix)
Ginkgo (Folia)
Ginseng (Radix)
Hederae helic. (Herba)
Hippocastani (Semen)
Liquiritiae (Radix)
Orthosiphonis (Folia)
Passiflorae (Herba)
Rauwolfiae (Radix)
Salicis (Cortex)
Solidaginis (Herba)
Tiliae (Flores)
Vitis vinifera (Folia, Fructus).

6. Procédé selon l'une quelconque des revendications 1 à 5, **caractérisé en ce que** la phase lipophile est d'origine animale, végétale, minérale ou synthétique et est en particulier non toxique, non aisément inflammable, non explosive et non volatile et est sélectionnée de préférence à partir du groupe comprenant:
- des graisses telles que le beurre de cacao, le gras de coco;
- des huiles telles que des huiles neutres, l'huile de tournesol, l'huile de noix de coco fractionnée telle que le Miglyol;
- des cires telles que les stéarines, l'huile de jojoba, la cire d'abeilles, l'huile de blanc de baleine, la cire de carnauba;
- des paraffines, y compris les vaselines;
- des lipoïdes; et
- des stérols,
tous ces composés, individuellement ou en mélanges, devant répondre de préférence aux exigences/définitions reprises dans le Codex allemand, DAB, ou dans la Pharmacopée britannique, BP, ou encore au Food Chemical Codex, FCC, aux Etats-Unis d'Amérique, respectivement doivent correspondre à ces exigences/définitions.

7. Procédé selon l'une quelconque des revendications 1 à 6, **caractérisé en ce que**, dans la première étape, le mélange des composants a lieu à une température telle qu'elle se situe entre le point de congélation et le point de fusion du mélange respectif, une température dans le domaine de la température ambiante à 70°C étant préférée.

8. Procédé selon l'une quelconque des revendications 1 à 7, **caractérisé en ce que**, dans la première étape, on mélange les composants l'un à l'autre pendant environ 1 heure, en particulier en secouant ou en agitant.

9. Procédé selon l'une quelconque des revendications 1 à 8, **caractérisé en ce que**, dans la deuxième étape, la séparation a lieu, soit au moyen d'une séparation de phases de deux phases liquides, soit au moyen d'une séparation de phases d'une phase liquide et d'une phase solide.

10. Procédé selon l'une quelconque des revendications 1 à 9, **caractérisé en ce que**, dans la deuxième étape, la séparation de deux phases liquides a lieu par séparation membranaire dans laquelle sont préférées des membranes présentant des ouvertures de pores dans le domaine de 0,001 à 1,0 micromètre, en particulier de 0,1 à 0,3 micromètre, en particulier des membranes en verre, en métal, en céramique et en matières synthétiques, par exemple en polypropylène ou en Téflon.

11. Procédé selon l'une quelconque des revendications 1 à 10, **caractérisé en ce qu'**on soumet la phase lipophile séparée dans la deuxième étape, qui contient les impuretés et/ou les résidus, à une distillation à la vapeur d'eau, on combine le distillat obtenu, qui contient des parfums et/ou des arômes lipophiles entraînables à la vapeur, comme tel ou après élimination préalable de l'eau avec la boisson purifiée obtenue à la fin de la troisième étape ou avec la préparation végétale purifiée obtenue à la fin de la troisième étape.

12. Procédé selon l'une quelconque des revendications 1 à 11, **caractérisé en ce qu'**on ajoute aux préparations végétales qui contiennent des alcaloïdes, avant de procéder à la première étape, au moins un acide physiologiquement acceptable, par exemple l'acide ascorbique, l'acide citrique, l'acide acétique en une quantité telle que l'on obtient une valeur de pH telle que les alcaloïdes sont présents sous forme de sels.
